# EUROPEAN PATENT APPLICATION

(11) **EP 1 541 129 A1**
(43) Date of publication of application: **15.06.2005**
(21) Application number: 03405893.3
(22) Date of filing: 12.12.2003
(51) Int. Cl.: A61K 9/00, A61K 31/43, A61K 31/424, A61P 31/04

(54) **Pharmaceutical effervescent formulation comprising amoxycillin and clavulanic acid**

(71) Applicant: Cimex AG, 4253 Liesberg (CH)
(72) Inventor: Scheer, Mathias, 79664 Wehr (DE)
(74) Representative: Arnold, Winfried

(57) **Abstract**

A pharmaceutical formulation comprising amoxycillin and clavulanate together with pharmaceutically acceptable ingredients, which is rapidly disintegrating and rapidly releasing the active ingredients into an aqueous fluid, showing a high bioavailability of the active ingredients which allows reduction of the clavulanate, methods for the preparation of such tablet and the use for oral treatment of bacterial infections.

## Description

### Field of the Invention

This invention relates to novel pharmaceutical formulations comprising amoxycillin and clavulanic acid, with rapid disintegrating and rapid dissolution properties. The active ingredients are readily dissolved or dispersed in aqueous fluids after disintegration of the formulation and are in this form easily orally administered. The presence of a buffer results in a surprising high bioavailability which allows reduction of the clavulanate content.

### State of the Art

Amoxycillin is a known beta-lactam antibiotic. Clavulanic acid is a known beta-lactamase inhibitor. Both compounds are used together in a synergistic manner in antibiotic formulations. Clavulanic acid, by inhibiting the enzyme beta-lactamase, serves to prolong the lifetime of amoxycillin in the patient by preventing early destruction thereof. In oral formulations amoxycillin is generally used in the form of its trihydrate, and clavulanic acid is generally used in the form of a pharmaceutically acceptable salt (hereinafter "clavulanate"), in particular the potassium clavulanate. For example GB 2005538 discloses formulations comprising amoxycillin trihydrate and potassium clavulanate. Such compositions are known and marketed under the trade name Augmentin®.

The Augmentin® tablet on the market is a film tablet formulation. Complete disintegration of this tablet in water at 37° C takes a relative long time, e.g. about 10 to 15 minutes.

For certain instances it is of advantage to provide oral pharmaceutical formulations which disintegrate very rapidly in aqueous fluids and, as a second requirement, also rapidly release the active material content in the stomach or in the intestine.

Existing sachets contain Augmentin® in the form of a powder, which is readily disintegrated in water and immediately forms a dispersion.

Another powder form is provided in so called "dry syrup" form. The powder is provided in a sufficiently large bottle, to which prior to use the prescribed amount of water is added to give multiple variable doses as a dispersion. The desired amount of the dispersion is taken orally by the patient according to his need. Such multiple variable "dry syrup" is particularly used for children. A drawback of the "dry syrup" powder is that the clavulanate is very sensitive to hydrolysis. After addition of water and upon storage of the dispersion the activity of clavulanate may be diminished or destroyed.

Amoxycillin trihydrate and clavulanate differ substantially with respect to their solubility in water. Potassium clavulanate is freely water-soluble (about 1 part of water for 1 part of solute), whereas amoxycillin trihydrate is only slightly soluble (about 100 to 1000 parts of solvent for 1 part of solute). In view of the sensitivity of clavulanate to hydrolysis its degradation may occur already in the aqueous fluid or between a relatively long period after oral administration and the contact with the acidic aqueous gastrointestinal fluids. Clavulanate has a maximum stability at pH 6.0 to 6.3.

Effervescent tablets or sachets are of advantage because of rapid disintegration. The time between addition of the formulation to water and swallowing of the dispersion or solution is therefore rather short.

US 6,051,254 discloses a pharmaceutical formulation comprising an amoxycillin hydrate, an effervescent couple and a β-lactamase inhibitor. Amoxycilin is not completely water soluble. In order to provide the required clear aqueous solution, an excess of alkaline component is essential to achieve a pH >8, to neutralise the acid components and completely solubilise the amoxycillin by salt formation. The effervescent couple is preferably based on citric acid and sodium bicarbonate or sodium glycine carbonate, or the like.

WO 98/35672 discloses pharmaceutical formulations, including tablets, comprising amoxycillin and clavulanate in a ratio of n:1 where n is a number of from 1 to 16, in form of a first set of granulates comprising amoxycillin and clavulanate, and a second set of granulates comprising amoxycillin and no clavulanate The tablet formulation may be a chewable, optionally effervescent, tablet. The effervescent couple may comprise a solid acid, for instance either citric acid, monosodium citrate, or sodium hydrogen citrate, and an alkali metal carbonate or bicarbonate, for instance sodium bicarbonate.

US 5,225,197 describes chewable tablets comprising a chewable base, selected from e.g. mannitol, sorbitol, dextrose, fructose, lactose or the like, a medicament, e.g. amoxycillin trihydrate in combination with potassium clavulanate, and an effervescent couple, e.g. anhydrous citric acid and sodium bicarbonate.

US 4,537,887 discloses pharmaceutical chewable tablets comprising amoxycillin trihydrate and potassium clavulanate in the range of 12:1 to 1:1, a pharmaceutical carrier and a desiccant in an effective amount of between 2% to 25% of the total weight of the formulated tablet.

Oral administration in aqueous solution or dispersion is, in particular, desirable for children, elderly or other patients, such as small animal patients.

The known pharmaceutical formulations comprising amoxycillin and clavulanate have certain disadvantages, insofar the pH of the solution or dispersion is not in the preferred range of about 6 which would provide the highest stability of the clavulanate. The early destruction of the clavulanate, also in the strong acidic conditions of the stomach, results in a lower bioavailability. Accordingly a relative large amount of the very expensive clavulanate is present in the formulations of prior art. High amounts of clavulanic acid cause side effects because they are not completely absorbed when passing the gastrointestinal tract [Martinez-Mir et al., Pharmakoepidemiology and Drug Safety, Vol. 5, p. 247-254 (1996)].

### Object of the Invention

Accordingly, from a clinical point of view the need exists to produce formulations with a lower amount of clavulanate, however without lowering the bioavailablity of clavulanic acid. This would allow to achieve the desired clinical effect simultaneously resulting in an improved benefit/risk ratio.

The original goal was to develop an effervescent formulation which is bio equivalent compared to a dry suspension of the originator. For this purpose an effervescent formulation containing a ratio of amoxycillin and clavulanic acid of 4:1 was manufactured. Surprisingly it has been found that the results in bio equivalence studies for clavulanic acid in the effervescent formulation were approx. 45% higher with regard to AUC (area under curve) and approx. 35% higher with regard to Cₘₐₓ (maximum concentration) when compared to the originator formulation.

It is an object of the present invention to avoid the drawbacks of formulations of prior art, and to produce an improved pharmaceutical formulation.

It is an object to produce a tablet or sachet which facilitates oral administration in aqueous solution or dispersion. The formulation should guarantee a higher stability of the clavulanic acid in the aqueous solution or dispersion and during gastric passage. Consequently a lower then normal amount of clavulanic acid is needed resulting in less side effects. A sufficient high bioavailability of the clavulanate is still provided. The formulation is rapidly dispersed in an aqueous fluid and orally administered, for example also by means of a spoon or drop by drop. Such formulations can be easily used for elderly patients, children, babies, as well as small and large animals.

The objects are achieved according to the present invention by providing a pharmaceutical formulation comprising amoxycilin, clavulanate, and a buffer. The formulation is readily disintegrated in aqueous fluids, in particular in water, within up to 5 minutes. The pH of the aqueous phase is in the range of about 6, where the clavulanic acid has the highest stability. The tablets may be divided for administration of lower amounts of active ingredients

The pharmaceutical formulations may be in the form of granulates which can be compressed into tablets, in particular effervescent tablets, or filled into sachets. In particular the effervescent formulations disintegrate fast, and rapidly and simultaneously releases the active ingredients amoxycillin and clavulanate into the aqueous fluid.

In order to provide simultaneous rapid release of the two active ingredients the formulation must warrant rapid disintegration and uniformity of the amoxycillin particles. These goals are achieved by the careful selection of the disintegrant, additional ingredients, and other parameters, such as method of production of the formulations, form and eventually hardness of the tablets. Another object is the easy and commercially advantageous and acceptable preparation of such formulations.

Another object is the use of such formulations for the production of tablets or sachets

Another object is the use of such formulations in combating infections in a patient in need thereof.

Another object is the reduction of side effects caused by non absorbed clavulanic acid.

Another object is to achieve a suprabioavailability of clavulanic acid compared to the state of the art.

The objects are achieved as set forth in the following detailed description, the examples and the claims.

### Detailed Description of the Invention

In a first embodiment the invention concerns a pharmaceutical formulation comprising a therapeutically effective amount of amoxycillin, a therapeutically effective amount of clavulanate, and a buffer.

The formulation is easily dispersible, rapidly disintegrating and, in particular, of effervescent nature.

The formulation can be manufactured into tablets or filled into sachets.

A pharmaceutical formulation according to the invention is composed of a first granulate comprising an effective amount of amoxycillin, a second granulate comprising a buffer, and a final blend comprising an effective amount of clavulanate,

An effervescent pharmaceutical formulation according to the invention is composed of a first granulate comprising an effective amount of amoxycillin, a second granulate, comprising an effective effervescent couple and a buffer, and a final blend comprising an effective amount of clavulanate,

In the formulation the active ingredient amoxycillin is preferably present in form of the trihydrate. The clavulanate is preferably present in form of potassium clavulanate.

The ratio of amoxycillin trihydrate and potassium clavulanate is between about 2:1 to 14:1, e.g. about 4:1, 7:1, 8:1, 9:1; 11:1, or preferably 10:1.

The formulations comprise e.g. about 583,40 mg of amoxycillin trihydrate and about 150 mg of potassium clavulanate (4:1), preferably 1004.60 mg of amoxycillin trihydrate and 109.41 mg of potassium clavulanate (10:1), or other ratios as shown in the examples.

The effervescent formulation, in the effervescent granulate, comprises a mixture of ingredients which release carbon dioxide upon contact with water. Such effervescent ingredients are in particular an alkali or alkaline earth metal carbonate or hydrogen carbonate, in particular sodium hydrogen carbonate, and an acid or acidic salt able to create carbon dioxide upon contact with an aqueous fluid. The acid may be a powdered anhydrous organic or inorganic pharmaceutically acceptable acid, in particular citric acid, monosodium citrate or preferably a mixture thereof. The mixture has the function of a buffer and provides a pH of about 6 when the formulation is dispersed in an aqueous fluid and also in the gastric fluid during gastric passage.

The pharmaceutical, in particular effervescent, formulation according to the invention comprises appropriate pharmaceutically acceptable further ingredients, such as excipients and coadjuvants selected from viscosity enlarging ingredients, fillers, desiccants, lubricants, diluents, binders, glidants, antifoaming agents, wetting agents, effervescent mixtures, sweeteners and flavourings.

For example, the amoxycillin comprising granulate comprises as pharmaceutically acceptable ingredients microcrystalline cellulose, crosspovidone, povidone K25, collidone, colloidal anhydrous silica, and optionally, a colouring agent, such as quinoline yellow.

The effervescent granulate comprise for example anhydrous citric acid, monosodium citrate, sorbitol, sodium hydrogen carbonate, a sweetener such as aspartam, povidone, Pharsil S 184, Tween 20, and a colouring agent, such as quinoline yellow.

The final blend comprises besides potassium clavulanate, for example microcrystalline cellulose, colloidal anhydrous silica, polyethylene glycol 6000, and the amoxycillin and effervescent granulates.

The pharmaceutical formulation, in particular the effervescent tablet or sachet, is disintegrating in aqueous fluids of room temperature within about 1 minute, in particular within 0.4 -1 minutes.

The pharmaceutical formulation, in particular the effervescent tablet or sachet, disintegrates in an aqueous beverage selected from water, fruit juice, syrup, and the like, and is administered orally.

After disintegration in water of 37° C, about 40 - 60 % of the amoxycillin trihydrate is released within 5 minutes and more then about 80% within 30 minutes.

Under the same conditions about 80 - 95 % of the potassium clavulanate is dissolved within about 5 minutes.

More particular the invention concerns a pharmaceutical dispersible formulation, which is composed of two granules, of which the first comprises an effective amount of amoxycillin trihydrate of from 500 mg to 1500 mg, the second granulate comprises an effective effervescent couple producing carbon dioxide if in contact with an aqueous fluid, and the final blend comprises an effective amount of potassium clavulanate of between 50 mg and 150 mg, whereby the final solution or dispersion in an aqueous fluid, in particular water, has a pH between about 5.6 and about pH 6.6.

The pH is controlled by an appropriate amount of a suitable pharmaceutically acceptable buffer in the effervescent granulate. Such buffers are pharmaceutical excipients or combinations thereof providing a buffer capacity. Such buffers are known in the art and comprise in particular mixtures of acids and alkali salts thereof. A preferred buffer in view of its pleasant taste is citric acid and monosodium citric acid in the ratio about 4:5.5.

.A preferred formulation comprises 1004.60 mg of amoxycillin trihydrate, 109.41 mg of potassium clavulanate, 400 mg of anhydrous citric acid 550 mg of monosodium citrate, 900 mg of sodium hydrogen carbonate, and pharmaceutical excipients, such as diluents, disintegrants, binders, glidants, sweeteners, antifoaming agents, wetting agents, lubricants, and flavouring agents.

Pharmaceutical, non-effervescent formulations, e.g. bilayered tablets, may also be prepared with pharmaceutical excipients in a similar manner as described hereinbefore, comprising the buffer, however without the carbon dioxide providing agent.

Pharmaceutical excipients are for instance: Diluents, e.g. microcrystalline cellulose, e.g. Avicel PH 105, disintegrants, e.g. crosspovidone, e.g. Kollidone CL-M, binders, e.g. povidone, e.g. Plasdone K25, glidants, e.g. colloidal anhydrous silica, e.g. Aerosil 200, sweeteners, e. g. sorbitol, antifoaming agents, e.g. simitone, wetting agents, e.g. polysorbate 20, lubricants, e.g. Macrogol 6000, and magnesium stearate, and flavouring agents, e. g. peach flavour.

The ingredients of the two granulates and the final blend are carefully selected to assure rapid effervescence, rapid disintegration and rapid simultaneous release of the two active ingredients into the aqueous fluid in the required pH range.

The granulates comprise the additional pharmaceutically acceptable ingredients independent from each other.

The disintegrant is of particular importance to assure a rapid disintegration of the tablet in aqueous fluids. The term "disintegrant" in this description refers to an agent, which increases the surface area of the tablet in water so that the tablet rapidly disintegrates into small particles. Polymers which have a high degree of disintegration power include, inter alia, cross linked sodium carboxymethylcellulose, cross-linked hydroxypropylcellulose, high molecular weight hydroxypropylmethylcellulose, carboxymethylamide, potassium methacrylatedivinylbenzene copolymer, polymethylmethacrylate, cross-linked polyvinylpyrrolidone, high-molecular weight polyvinyl alcohols, microcrystalline cellulose, and the like. Examples of such polymers include Methocel K4M® , Methocel E5® , Methocel E50® , Methocel E4M® , Methocel K15M® .

Particular examples of disintegrants are sodium starch glycolate, e. g. Explotab® , polymeric derivatives of acrylic acid, and preferably crosprovidone (an insoluble polyvinylpyrrolidone), e.g. PolyPolyplasdone® XL, or microcrystalline cellulose, e. g. Avicel® .

Sodium starch glycolate may be used in concentrations exceeding 5% by weight in relation to the total weight of the formulation, in particular about 9.5% to 17%.

Polymeric derivatives of acrylic acid may be used in amounts of between about 10% to 21% of the total formulation weight.

Crosspovidone may be used in a concentration of between about 4% and 16%, in particular about 4.15% in the amoxycillin formulation and about 15.5% in the clavulanate formulation of the total formulation weight.

Fillers or diluents facilitate compression of the powder and have an influence on the hardness of the tablet after compression. Such compounds comprise mannitol, microcrystalline cellulose, e. g. Avicel® PH101, in particular cellulose powder, e. g. Elcema® G 250, and silicon dioxide, e.g. Syloid® AL FP.

Fillers are used in amounts of about 5% to 10%, in particular 8.3% in the amoxycillin formulation and about 20% to 50%, in particular 39% in the clavulanate formulation of the total formulation weight.

Lubricants are excipients which reduce inter-particle friction inside the tablet and reduce the reaction forces appearing on the wall of the matrix. Lubricants are for example talcum, stearyl fumarate, polyethylene glycol, salts of benzoic acid, such as the sodium or lithium salt, and in particular magnesium stearate. Lubricants may be used in amounts of between about 0.1% and 1%, in particular about 0.2% in the amoxycillin formulation and about 0.65% in the clavulanate formulation in relation to the total formulation weight.

Further ingredients may include binders, such as Polyplasdone K29-32® , polyethylene glycol 6000, e. g. Magrocol®, compression aids, and the like.

Flavouring agents have for example the taste of a natural fruit, such as orange, lemon, apple, strawberry, vanilla, or of a herb, for example peppermint, or of broiled or fried meat, such as extracts from liver or yeast.

Sweetening agents are for example, saccharin, aspartame, cyclamate and mixtures thereof. The flavouring and sweetening agents and the amounts should be adjusted to the patient to be treated.

The pharmaceutical formulations of the invention essentially include a pH modifying agent, in particular a pH buffer, which preferably is present in the effervescent granulate, possibly also in the final blend. A preferred buffer adjusts the pH of the final solution or dispersion to a pH of about 6 and is in particular a mixture of citric acid and monosodium citrate.

Colouring agents serve to give a pleasant view and/or to optically distinguish between the two formulations. Such agents are for example tartrazin (E102), crinoline yellow (E104) and yellow orange (E110)

Suitably such a rapid release formulation may comprise around 30 - 70 % (all percentages given herein are on a weight percentage basis unless otherwise stated) e.g. 50 - 60% of active material content, around 10 - 30% of disintegrants, fillers, lubricants, compression aids, viscosity enlarging ingredients, tablet binders, colouring agents, viscosity enlarging ingredients, tablet binders, colouring agents, flavouring and sweetening agents, and the like.

Preferred ingredients in the amoxycillin granulate are for example amoxycillin trihydrate, microcrystalline cellulose, crosspovidone, and povidone.

Preferred ingredients of the effervescent granulate are for example citric acid, monosodium citrate, sorbitol, and sodium hydrogen carbonate. Further ingredients may be aspartame, sodium cyclamate, povidone, simeticone, and polysorbate 20.

Preferred ingredients in the final blend clavulanate formulation are for example potassium clavulanate, microcrystalline cellulose, magrogol 6000, silicon dioxide, colloidal and anhydrous silica, magnesium stearate, and a flavour, e.g. peach or orange flavour.

The term "about" as used hereinbefore and hereinafter in connection with weights or other measures should be interpreted to mean plus or minus 10%.

The pharmaceutical formulation according to the invention may be pressed into tablets or filled into sachets according to conventional methods.

### The Manufacture of the Pharmaceutical Formulations

In a second embodiment the invention concerns methods for the manufacture of pharmaceutical formulations by using wet or dry granulating techniques. A specific method is e. g. characterised in
a) preparing an amoxycillin comprising granulate by homogeneously mixing amoxycillin with the appropriate additional pharmaceutically acceptable excipients and coadjuvants, granulating the mixture, drying the granulates and passing the granulates through a suitable sieve,
b) preparing an effervescent granulate by homogeneously mixing the effervescence with the additional pharmaceutically acceptable excipients and coadjuvants, granulating the mixture, drying the granulates and passing the granulates through a suitable sieve, and
c) preparing a final blend by mixing the clavulanate with a predried mixture of the appropriate additional pharmaceutically acceptable excipients and coadjuvants, adding the amoxycillin granulate and then the effervescent granulate, and
d) compressing the mixture into tablets or filling into sachets or bottles.

More particularly, the following steps are performed:
a1) premixing the ingredients of the amoxycillin granulate,
a2) preparing a granulation liquid consisting of anhydrous alcohol and purified water,
a3) granulating by distributing the granulation liquid on the blend of step a1) and subsequent kneading,
a4) drying the granules,
a5). sieving of the granules,
b6) blending the ingredients of the effervescent granules,
b7) preparing a granulation liquid consisting of ethanol, purified water and polysorbat 20,
b8) granulating by distributing the granulation liquid on the blend of step b6) and add simeticone,
b9) drying the granules,
b10) sieving of the granules and filling into stainless steel drum with desiccant,
c11) pre-drying microcrystalline cellulose and silicone dioxide,
c12) pre-drying macrogol 6000 and peach flavour,
c13) prepare a clavulanic acid mixture by mixing potassium clavulanate, microcrystalline cellulose, and colloidal anhydrous silica and passing through a sieve and mixing again, adding silicon dioxide, microcrystalline cellulose and the amoxycillin granulate of step c10), mixing and passing through a sieve and mixing again,
c14) finally blend the amoxycillin granulate, the effervescent granulate and the clavulanic acid mixture of step c13), adding macrogol 6000, peach flavour, and magnesium stearate and mixing again,
d15) compressing the blend of step 14 into tablets or filling it into sachets or bottles, and
d16) finally packaging.

Typically the active material content (may also be amoxycillin powder), disintegrants, fillers, lubricants, viscosity enlarging ingredients, tablet binders, colouring agents, viscosity enlarging ingredients, tablet binders, colouring agents, flavouring and sweetening agents, when used, are mixed, then lubricants and compression aids are added. The complete mixture, in particular when amoxycillin powder is used, may then be compressed under high pressure in the tablet press. Roller compaction may be used to form granules for tabletting. Alternatively wet granulation may be used, isopropanol or in particular ethanol/ water mixture, being a suitable solvents. A suitable wet granulating aid is Polyplasdone K29-32® .

Clavulanic acid and its derivatives are extremely moisture sensitive. Potassium clavulanate is the most stable pharmaceutically acceptable derivative. Therefore formulations containing derivatives of clavulanic acid, such as potassium clavulanate, should be made up under dry conditions, preferably at 20% relative humidity or less at 20° C. The ingredients of the formulation should be pre-dried where appropriate. Formulations of the invention including derivatives of clavulanic acid should be stored in containers which are sealed against the ingress of atmospheric moisture.

The tablet formulations and the granules of the invention may contain up to the maximum permitted daily dose of amoxycillin and clavulanate per unit dose or portions thereof. The formulations may contain for example around 1000 mg of amoxycillin trihydrate and around 109 mg of potassium clavulanate. Suitable unit dose tablets or granules of the invention may contain the following nominal weights (mg) of amoxycillin : clavulanate (expressed as free acid equivalent): ratio 7:1 (e.g. 875:125; 500:71.428; or 250:35.71); ratio 8:1 (e.g. 875:109.375; 500:62.5; or 250:31.35), 9:1 (e.g. 1004.60:125.11), 11:1 (e.g. 1004.60:102.46) or ratio 4:1 (e.g. 200:50; or 400:100. Preferred are 10:1 formulations (e.g. 1004.60:109.41). But also any other absolute amounts of these drug substances or both drug substances in any other ratio are envisaged.

Small doses for children, babies or small animals have a weight ratio of amoxycillin : clavulanate from 4:1 to 14:1, and the weights (mg) may be about 40:10, e.g. also for cats; 200:50, e.g. for small dogs, or 400:100 large dogs.

Large animals, such as horses, cows, pigs and the like need higher dosages, which can be achieved by application of more than one tablet.

Preferably effervescent dispersible tablets and granules according to the invention are those described in the Examples. The hardness of the effervescent tablet is above 50, and may be about 100 to 300 Newton, preferable about 180 Newton.

The form of the tablets is one of the usual round, spherical, block or oblong forms.

Suitably the tablet formulations of the invention may be formed by known compression tabletting techniques, for example using a known multi-layer tabletting press. Suitably a dry densification process may be used, e.g. briquetting. The Tablets may have any conventional structure.

In another embodiment the invention concerns a method of use of a pharmaceutical formulation as set forth above for the manufacture of a medicament for the treatment of bacterial infections.

In another embodiment the invention concerns a method of orally treating bacterial infections of a host, comprising administering to said host an aqueous fluid prepared from an effervescent pharmaceutical formulation in form of a tablet or granulate according to the invention.

In a further embodiment, the invention provides the use of a pharmaceutical formulation in form of a tablet or granulate as an active therapeutic in the treatment of bacterial infections of a host in need thereof.

The effervescent or dispersible tablets or the content of the sachets of the invention are dispersed in water or another aqueous fluid at room temperature, e.g. between 15 to 25° C, and administered orally inn the form of a dispersion. The amount of fluid is not critical. In general an amount for easy swallowing is taken. Children and babies are conveniently treated with a disintegrated dispersion of a tablet in water, a syrup, or a fruit juice, a tea, or any other pharmaceutically acceptable water containing fluid, eventually spoon by spoon. For animals the tablets may be added to the food, or disintegrated in water and this form added to the food or injected into the mouth by means of a pipette.

For oral administration the amount of the aqueous fluid for one tablet is from e.g. 50 to 100 ml to achieve a concentration of the amoxycillin of about 0.041% to 2%, preferably about 0.04% to 0.875%, and of clavulanate of about 0.01% to 0.3%, preferably 0.01% to 0.125%, +/- 10 %.

The pharmaceutical formulations of this invention immediately and simultaneously provide high plasma levels of amoxycillin and clavulanate after oral administration.

In a further embodiment the invention provides the use of the pharmaceutical formulations for producing a pharmaceutical package A pharmaceutical package may be any of a suitable package in this field, such as a filled up glass bottle, aluminum tube, optionally equipped with a desiccant, or a suitable aluminum strip.

The concept of the supra-bioavailability of clavulanic acid in effervescent formulations is presented in the examples. The results consistently show a bioavailability of clavulanic acid in these formulations which is approx. 40% higher than in conventional formulations. The reason is attributed to the fact that the buffered composition of the pharmaceutical formulation reduces the decomposition of the clavulanic acid during the gastric passage.

Following is a short description of the accompanying drawings:

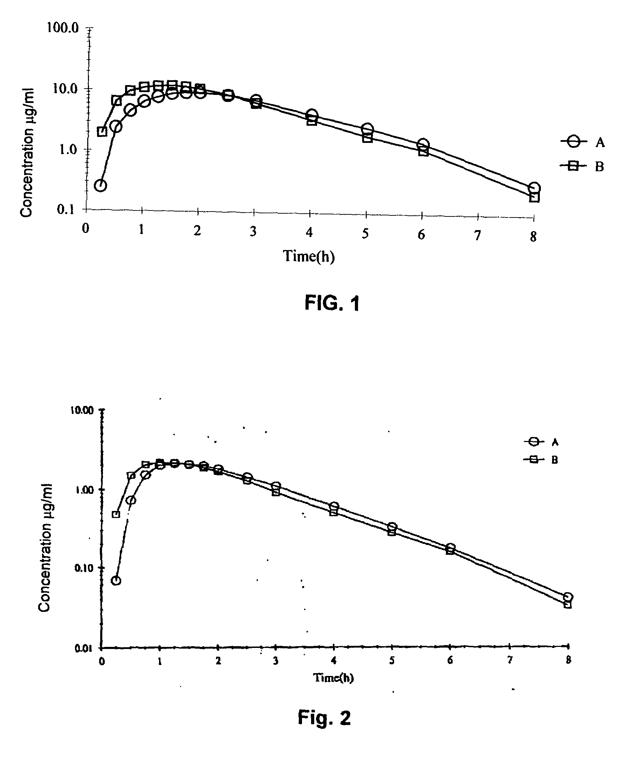

Fig. 1 shows a semi logarithmic plot of mean plasma concentrations of amoxycilin versus time curves after administration of a commercial reference compound A (7:1 of Example 2), and a test compound B (10:1 of Example 2) to healthy, adult human males under fasting conditions.
Fig. 2 shows a semi logarithmic plot of mean plasma concentrations of clavulanic acid versus time curves after administration of a commercial reference compound A (7:1 of Example 2) and a test compound B (10:1 of Example 2) to healthy, adult human males under fasting conditions.

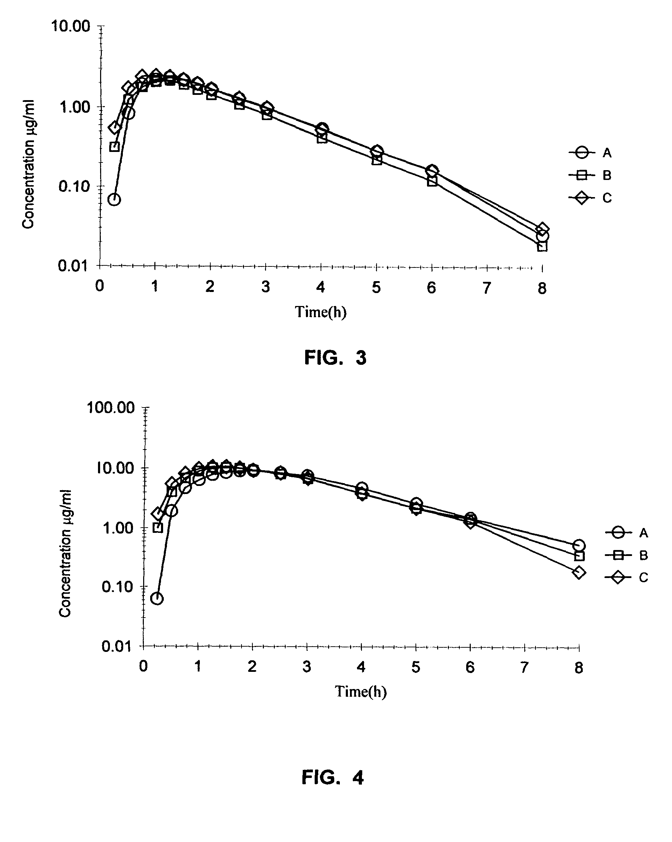

FIG. 3 shows a semi logarithmic plot of mean plasma concentrations of amoxycillin versus time curves after administration of a commercial reference compound A (curve A: 7:1) and an effervescent tablet containing 875/79.6 mg of amoxycillin/clavulanic acid 11:1 (curve B, Example 3) and an effervescent tablet of amoxycillin/clavulanic acid containing 875/97.2 mg of amoxycillin/clavulanic acid 9:1 (curve C, Example 4) to healthy, adult human males under fasting conditions.
Fig. 4 shows a semi logarithmic plot of mean plasma concentrations of clavulanic acid versus time curves after administration of a commercial reference compound A (curve A: 7:1) and an effervescent tablet containing 875/79.6 mg of clavulanic/clavulanic acid 11:1 (curve B, Example 3) and an effervescent tablet of clavulanic/clavulanic acid containing 875/97.2 mg of amoxycillin/clavulanic acid 9:1 (curve C, Example 4) to healthy, adult human males under fasting conditions.

In the Figures compounds A, curves A, correspond to Augmentan film tablet containing 875/125 mg of clavulanic/clavulanic acid 7:1, manufactured by SmithKline Beecham, Germany

The following examples serve to further characterise the invention, however, should not be construed as a limitation thereof.

### Example 1. Preparation of effervescent formulations with 583,40 mg of amoxycillin trihydrate and 150.00 mg of potassium clavulanate (4:1)

The components of the amoxycillin/clavulanic acid 500/125 mg effervescent formulation are given in Table 1:

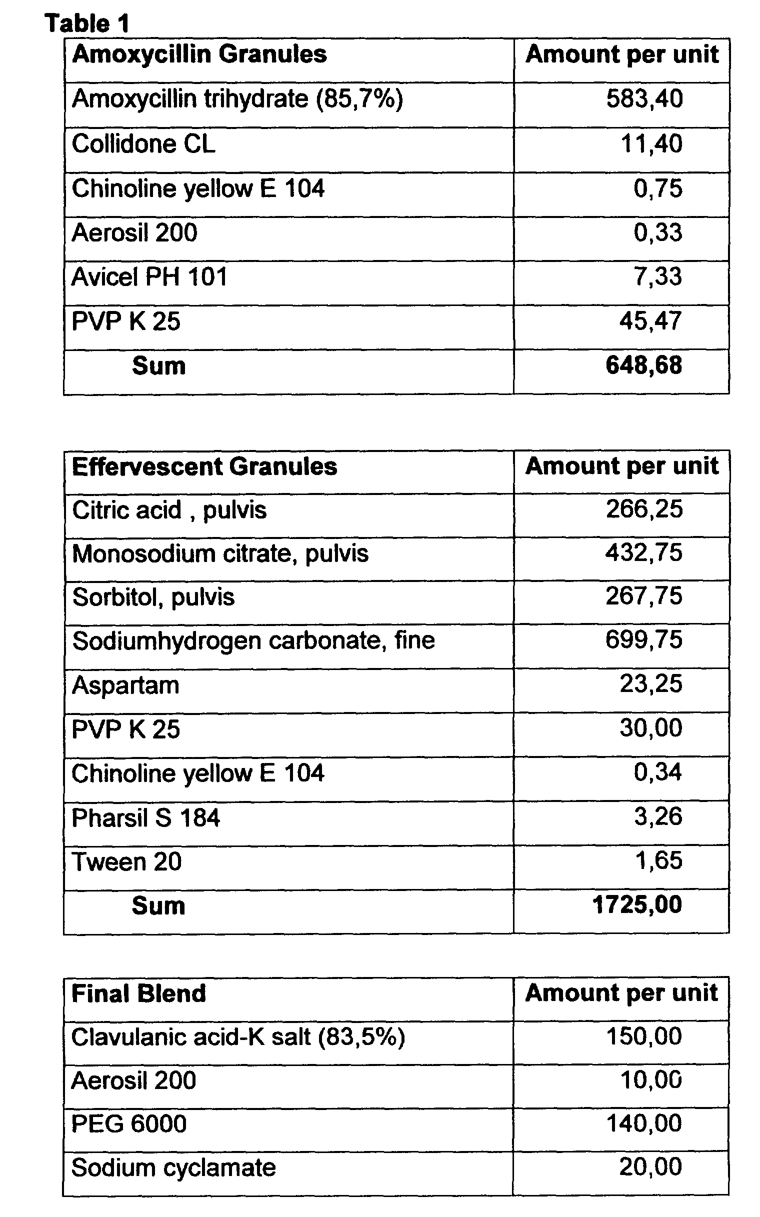

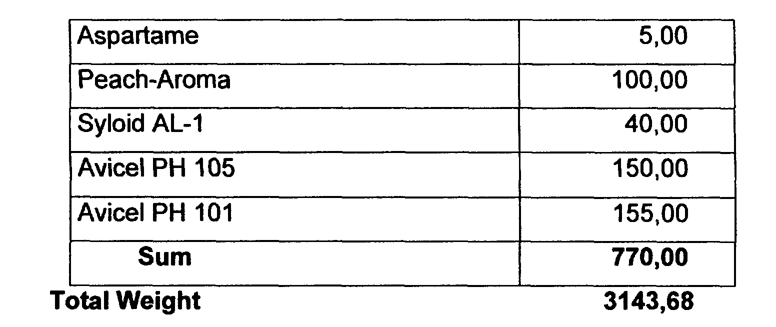

### Manufacturing Description

### Amoxycillin Granulate

Amoxycillin trihydrate, microcrystalline cellulose, crospovidone, povidone K25, quinoline yellow, collidone CL and colloidal anhydrous silica are mixed until a homogeneous blend is achieved (premix).

A mixture of anhydrous ethanol and purified water (granulation liquid) is added to the premix for granulation and subsequently kneaded until a well structured granulate is obtained. The granules are dried and sieved.

### Effervescent Granulate

Anhydrous citric acid, monosodium citrate, sorbitol, sodium hydrogen carbonate, aspartame, povidone and chinoline yellow are weighed and mixed (premix).

Pharsil S 184 and Tween 20 are dissolved in a mixture of anhydrous ethanol and purified water (granulation liquid).

The granulation liquid is added to the premix for granulation. The obtained granules are dried, sieved and filled into stainless steel drums equipped with desiccant.

### Final blend

Microcrystalline cellulose (Avicel PH 101 and PH 105), silicon dioxide, PEG 6000 and peach flavour are pre-dried and stored in tight containers.

Potassium clavulanate, microcrystalline cellulose and colloidal anhydrous silica are mixed until a homogeneous blend is obtained. Subsequently, silicon dioxide, microcrystalline cellulose and a part (1/10) of amoxycillin granulate are added. The mixture is passed through a sieve and is mixed.

The rest of the amoxycillin granulate (9/10), the effervescent granulate (step 10) and the mixture of clavulanic acid are mixed. PEG 6000, peach flavour, sodium cyclamate and aspartame are added and mixed again. Subsequently the mixture is compressed into tablets.

The pH of the aqueous dispersion of the tablet in 200 ml of water is about 6.

### Bio equivalence Study of the formulation of Example 1

### Objective:

It was the objective to compare the pharmacokinetic profile of the effervescent amoxycillin and clavulanic acid formulation described in Example 1 (ratio amoxycillin:clavulanic acid 4:1) relative to that of a commercial product ('Augmentan' dry suspension, ratio amoxycillin:clavulanic acid 4:1) in healthy, adult, male, human subjects under fasting conditions, and to assess the bio equivalence.

The study design was an open label, randomized, balanced, two-treatment, two-period, two- sequence, crossover, single dose, comparative, oral bioavailability study in healthy, adult, male, human subjects under fasting conditions.

The allocation of the subjects to the treatment sequence was done as per a randomisation schedule. Serial blood sampling before dosing and up to 8 hours after drug administration was performed. Plasma concentrations of amoxycillin and clavulanic acid were analysed by a validated HPLC method. Calculation of pharmacokinetic parameters using plasma amoxycillin and clavulanic acid concentration time profiles was done by non-compartmental model. Statistical comparison of the pharmacokinetic parameters of the two formulations was done to assess bio equivalence.

Bio equivalence was to be concluded if the 90% confidence intervals (Cl) for In-transformed pharmacokinetic parameters for amoxycillin and clavulanic acid fell with in the range as shown in Table 2:

**Table 2**

| **Drug** | **Parameters** | **Range of In-transformed 90% Cl** |
|---|---|---|
| Amoxycillin | Cₘₐₓµg/ml | 0.75-1.33 (75.00 - 133.00 %) |
| | AUC₀₋₈¹⁾ | 0.80 -1.25 (80.00-125.00 %) |
| | AUC_{0-∞}²⁾ | 0.80 - 1.25 (80.00 - 125.00 %) |
| Clavulanic Acid | Cmax | 0.70-1.43 (70.00 - 143.00 %) |
| | AUC₀₋₈¹⁾ | 0.75 - 1.33 (75.00-133.00 %) |
| | AUC_{0-∞}²⁾ | 0.75 - 1.33 (75.00-133.00 %) |

| | | |
|---|---|---|
| ¹⁾ Area under curve from 0-8 hours; µgh/ml | | |
| ²⁾ Area under curve from 0-∞ hours, µgh/ml | | |

### Results:

It has surprisingly been found that the results for clavulanic acid in the effervescent formulation were approx. 45% higher with regard to AUC and approx. 35% higher with regard to Coax when compared to the originator formulation:

**Table 3:**

| **Clavulanic acid (34 Subjects)** | | |
|---|---|---|
| **Parameters** **(Units)** | **Point estimator** **(Test/Reference)** | **Ln transformed 90%** **Confidence Interval** |
| Cₘₐₓµg/ml | 1.32 | 1.19 - 1.48 |
| AUC₀₋₈¹⁾ | 1.45 | 1.29 - 1.63 |
| AUC_{0-∞}²⁾ | 1.43 | 1.28 - 1.60 |

The results for amoxycillin were found to be in the predefined acceptance ranges for AUC and Cₒₐₓ:

**Table 4:**

| **Amoxycllin (34 Subjects)** | | |
|---|---|---|
| **Parameters** **(Units)** | **Point estimator** **(Test/Reference)** | **Ln transformed 90%** **Confidence Interval** |
| Cₘₐₓµg/ml | 1.02 | 0.96 - 1.08 |
| AUC₀₋₈¹⁾ | 1.01 | 0.98 - 1.04 |
| AUC_{0-∞} ²⁾ | 1.01 | 0.98-1.04 |

The results of Example 1 led to a formulation with a ratio of amoxycillin and clavulanic acid of 10:1 as shown in Example 2:

### Example 2. Preparation of effervescent formulations with 1004.60 mg of amoxycillin trihydrate and 109.41 mg of potassium clavulanate (10:1)

White to pale yellow, round effervescent tablets or sachets each containing 1004.6 mg of amoxycillin trihydrate (corresponding to 875 mg of amoxycillin) and 104.2 mg of potassium clavulanate (corresponding to 87.5 mg of clavulanic acid) are produced as follows. The ingredients for the two granulates and the final blend of one tablet or sachet are given in Table 5.

**Table 5:**

| **Amoxycillin granules** | **Amount per unit** |
|---|---|
| Amoxycillin trihydrate | 1004.60 mg |
| Microcrystalline cellulose | 24.40 mg |
| Crospovidone | 26.00 mg |
| Povidone | 40.00 mg |
| Colloidal anhydrous silica | 5.00 mg |
| Ethanol, anhydrous | (192.00 mg) |
| Purified water | (88.00 mg) |
| **Amount** | **1100.00 mg** |

| **Effervescent granules** | **Amount per unit** |
|---|---|
| Citric acid, anhydrous | 400.00 mg |
| Monosodium citrate | 550.00 mg |
| Sorbitol | 326.00 mg |
| Sodium hydrogen carbonate | 900.00 mg |
| Aspartame | 35.00 mg |
| Sodium cyclamate | 20.00 mg |
| Povidone | 40.00 mg |
| Simeticone | 2.00 mg |
| Polysorbate 20 | 2.00 mg |
| Ethanol, anhydrous | (96.00 mg) |
| Purified water | (4.00 mg) |
| **Amount** | **2275.00 mg** |

| **Final Blend** | **Amount per unit** |
|---|---|
| Potassium clavulanate | 109.41 mg |
| Microcrystalline cellulose | 109.41 mg |
| Microcrystalline cellulose | 145.18 mg |
| Macrogol 6000 | 140.00 mg |
| Silicon dioxide | 40.00 mg |
| Colloidal anhydrous silica | 10.00 mg |
| Magnesium stearate | 1.00 mg |
| Peach flavour | 100.00 mg |
| Amount | 655.00 mg |
| **Total amount per tablet or sachet** | **4030.00 mg** |

### Manufacturing Description

### Amoxycillin Granulate

Amoxycillin trihydrate, microcrystalline cellulose, crospovidone, povidone K25 and colloidal anhydrous silica are mixed until a homogeneous blend is achieved (premix).

A mixture of anhydrous ethanol and purified water (granulation liquid) is added to the premix for granulation and subsequently kneaded until a well structured granulate is obtained. The granules are dried and sieved.

### Effervescent Granulate

Anhydrous citric acid, monosodium citrate, sorbitol, sodium hydrogen carbonate, aspartame, povidone and sodium cyclamate are weighed and mixed (premix).

Polysorbate 20 is dissolved in a mixture of anhydrous ethanol and purified water (granulation liquid).

The granulation liquid and the simeticone is added to the premix for granulation. The obtained granules are dried, sieved and filled into stainless steel drums equipped with desiccant.

### Final blend

Microcrystalline cellulose (Avicel PH 101 and PH 105), silicon dioxide Macrogol 6000 and peach flavour are pre-dried and stored in tight containers.

Potassium clavulanate, microcrystalline cellulose and colloidal anhydrous silica are mixed until a homogeneous blend is obtained.. Subsequently, silicon dioxide, microcrystalline cellulose and a part (1/10) of amoxycillin granulate are added. The mixture is passed through a sieve and is mixed.

The rest of the amoxycillin granulate (9/10), the effervescent granulate (step 10) and the mixture of clavulanic acid are mixed. Macrogol 6000, peach flavour and magnesium stearate are added and mixed again. Subsequently the mixture is compressed into tablets or filled into sachets.

The pharmacokinetic profile of the effervescent amoxycillin and clavulanic acid formulation described in Example 2 (ratio amoxycillin:clavulanic acid 10:1) relative to that of a commercial product ('Augmentan' tablets, ratio amoxycillin:clavulanic acid 7:1) in healthy, adult, male, human subjects under fasting conditions was compared, and the bio equivalence assessed. The same methodology was employed as in the study described above. The results are compiled in Table 6

**Table 6:**

| **Clavulanic acid (36 Subjects)** | | |
|---|---|---|
| **Parameters** **(Units)** | **Point estimator** **(Test/Reference)** | **Ln transformed 90%** **Confidence Interval** |
| Cₘₐₓµg/ml | 107.7 % | 94.23-123.05 % |
| AUC₀₋₈¹⁾ | 106.1 % | 93.53-120.41 % |
| AUC_{0-∞}²⁾ | 105.5% | 93.54-118.92% |

It was confirmed that the bioavailability of clavulanic acid in the effervescent formulation is approx. 40% higher than in the tablet formulation. As a result, the bio equivalence of clavulanic acid is given even though the ratio of clavulanic acid: amoxycillin is 1:10 instead of 1:7.

The tablet has a height of 6.5 - 7.5 mm and a diameter of 24.8 - 25.3 mm. The resistance to crushing (n=5) is ≥ 50 N. The disintegrating time is below ≤ 5 minutes in water at 20° C.

A pH of about 6 results when the tablet is dispersed in 200 ml of water. Again, the results for amoxycillin were found to be in the predefined acceptance ranges as shown in Table 7:

**Table 7:**

| **Amoxycillin (36 Subjects)** | | |
|---|---|---|
| **Parameters** **(Units)** | **Point estimator** **(Test/Reference)** | **Ln transformed** **90% Confidence Interval** |
| Cₘₐₓµg/ml | 124.0 | 116.36-132.31 |
| AUC₀₋₈¹⁾ | 111.9 | 104.22-120.55 |
| AUC_{0-∞}²⁾ | 110.8 | 103.84-119.16 |

### Example 3: Effervescent formulation containing 1004.60 mg trihydrate-trihydrate and 102.46 mg potassium clavulanate (11:1)

Following the Manufacturing Descriptions of Example 1 or 2 the components of Table 8 are manufactured.

**Table 8**

| **Amoxycillin granules** | **Amount per unit** |
|---|---|
| Amoxycillin trihydrate | 1004.60 mg |
| Microcrystalline cellulose | 24.40 mg |
| Crospovidone | 26.00 mg |
| Povidone | 40.00 mg |
| Colloidal anhydrous silica | 5.00 mg |
| Ethanol, anhydrous | (192.00 mg) |
| Purified water | (88.00 mg) |
| **Sum** | **1100.00 mg** |

| **Effervescent granules** | |
|---|---|
| Citric acid, anhydrous | 400.00 mg |
| Monosodium citrate | 550.00 mg |
| Sorbitol | 326.00 mg |
| Sodium hydrogen carbonate | 900.00 mg |
| Aspartame | 35.00 mg |
| Sodium cyclamate | 20.00 mg |
| Povidone | 40.00 mg |
| Simeticone | 2.00 mg |
| Polysorbate 20 | 2.00 mg |
| Ethanol, anhydrous | (96.00 mg) |
| Purified water | (4.00 mg) |
| **Sum** | **2275.00 mg** |

| **Final Blend** | **Amount per unit** |
|---|---|
| Potassium clavulanate | 102.46 mg |
| Microcrystalline cellulose | 109.41 mg |
| Microcrystalline cellulose | 145.18 mg |
| Macrogol 6000 | 140.00 mg |
| Silicon dioxide | 40.00 mg |
| Colloidal anhydrous silica | 10.00 mg |
| Magnesium stearate | 1.00 mg |
| Peach flavour | 100.00 mg |
| **Sum** | **648.05 mg** |
| **Total amount per tablet** | **4023.05 mg** |

### Results of the Bioequivalence Study of Example 3

The pharmacokinetic profile of the effervescent amoxycillin and clavulanic acid formulation described in Example 3 (ratio amoxycillin:clavulanic acid 11:1, Test B) relative to that of a commercial product ('Augmentan' tablets, ratio amoxycillin:clavulanic acid 7:1, Reference A) in healthy, adult, male, human subjects under fasting conditions was compared, and the bioequivalence to assessed. The same methodology was employed as in the study described above. The following results were obtained:

**Table 9:**

| **Clavulanic acid (12 Subjects)** | | |
|---|---|---|
| **Parameters (Units)** | **Point estimator** **(Test/Reference)** | **Ln transformed** **90% Confidence Interval** |
| Cₘₐₓµg/m) | 94.4% | 81.8-108.9 % |
| AUC₀₋₈¹⁾ | 91.5% | 77.4-108.3 % |
| AUC_{0-∞}²⁾ | 91.3% | 77.7-107.2 % |

**Table 10:**

| **Amoxycillin (12 Subjects)** | | |
|---|---|---|
| **Parameters (Units)** | **Point estimator** **(Test/Reference)** | **Ln transformed** **90% Confidence** |
| Cₘₐₓµg/ml | 115.7 % | 99.1-135.2 % |
| AUC₀₋₈¹⁾ | 105.1 % | 92.0-120.2 % |
| AUC_{0-∞}²⁾ | 103.7% % | 91.5-117.6% |

### Example 4: Effervescent formulation containing 1004.60 mg amoxycillin-trihydrate/125.11 mg potassium clavulanate (9:1)

Following the Manufacturing Descriptions of Example 1 or 2 the components of Table 11 are manufactured.

**Table 11**

| **Amoxycillin granules** | **Amounts per Unit** |
|---|---|
| Amoxycillin trihydrate | 1004.60 mg |
| Microcrystalline cellulose | 24.40 mg |
| Crospovidone | 26.00 mg |
| Povidone | 40.00 mg |
| Colloidal anhydrous silica | 5.00 mg |
| Ethanol, anhydrous | (192.00 mg) |
| Purified water | (88.00 mg) |
| **Sum** | **1100.00 mg** |

| **Effervescent granules** | |
|---|---|
| Citric acid, anhydrous | 400.00 mg |
| Monosodium citrate | 550.00 mg |
| Sorbitol | 326.00 mg |
| Sodium hydrogen carbonate | 900.00 mg |
| Aspartame | 35.00 mg |
| Sodium cyclamate | 20.00 mg |
| Povidone | 40.00 mg |
| Simeticone | 2.00 mg |
| Polysorbate 20 | 2.00 mg |
| Ethanol, anhydrous | (96.00 mg) |
| Purified water | (4.00 mg) |
| **Sum** | **2275.00 mg** |

| **Final Blend** | **Amount per unit** |
|---|---|
| Potassium clavulanate | 125.11 mg |
| Microcrystalline cellulose | 109.41 mg |
| Microcrystalline cellulose | 145.18 mg |
| Macrogol 6000 | 140.00 mg |
| Silicon dioxide | 40.00 mg |
| Colloidal anhydrous silica | 10.00 mg |
| Magnesium stearate | 1.00 mg |
| Peach flavour | 100.00 mg |
| **Sum** | **670.70 mg** |
| **Total amount per formulation** | **4045.70 mg** |

### Results of the Bioequivalence Study of Example 4

The pharmacokinetic profile of the effervescent amoxycillin and clavulanic acid formulation described in Example 4 (ratio amoxycillin:clavulanic acid 9:1, Test C) relative to that of a commercial product ('Augmentan' tablets, ratio amoxycillin:clavulanic acid 7:1, Reference A) in healthy, adult, male, human subjects under fasting conditions was determined to assess the bioequivalence. The same methodology was employed as in the study described above. The results are compiled in Tables 12 and 13:

**Table 12:**

| **Clavulanic acid (12 Subjects)** | | |
|---|---|---|
| **Parameters (Units)** | **Point estimator** **(Test/Reference)** | **Ln transformed** **90% Confidence Interval** |
| Cₘₐₓµg/ml | 111.5 % | 96.7-128.7 % |
| AUC₀₋₈¹⁾ | 113.2 % | 95.7-134.0 % |
| AUC_{0-∞}²⁾ | 112.1 % | 95.5-131.7 % |

**Table 13:**

| **Amoxycillin (12 Subjects)** | | |
|---|---|---|
| **Parameters (Units)** | **Point estimator** **(Test/Reference)** | **Ln transformed** **90% Confidence Interval** |
| Cₘₐₓµg/ml | 118.3 % | 101.3-138.2 % |
| AUC₀₋₈¹⁾ | 108.1 % | 94.6-123.54 % |
| AUC_{0-∞}²⁾ | 107.5% | 94.8-121.90 % |

## Claims

1. A pharmaceutical formulation comprising a therapeutically effective amount of amoxycillin, a therapeutically effective amount of clavulanate, and a buffer.

2. A pharmaceutical formulation according to claim 1, which is an effervescent formulation.

3. A pharmaceutical formulation according to claim 1 or 2, which is dispersible and rapidly disintegrating.

4. A pharmaceutical formulation according to claim 1 or 2 in form of a tablet.

5. A pharmaceutical formulation according to claim 1 or 2 filled up in form of granules.

6. A pharmaceutical formulation according to any one of claims 1 to 5 composed of a first granulate comprising an effective amount of amoxycillin, a second granulate, comprising an effective effervescent couple and a buffer, and a final blend comprising an effective amount of clavulanate,

7. A pharmaceutical formulation according to any one of claims 1 to 6, wherein amoxycillin is present in form of the trihydrate.

8. A pharmaceutical formulation according to any one of claims 1 to 6, wherein clavulanate is present in form of potassium clavulanate.

9. A pharmaceutical formulation according to any one of claims 1 to 8, wherein the ratio of amoxycillin trihydrate and potassium clavulanate is between about 2:1 to 14:1.

10. A pharmaceutical formulation according to any one of claims 1 to 9, wherein the ratio of amoxycillin trihydrate and potassium clavulanate is about 7:1, 8:1, 9:1. or 11:1.

11. A pharmaceutical formulation according to any one of claims 1 to 9, wherein the ratio of amoxycillin trihydrate and potassium clavulanate is about 10:1.

12. A pharmaceutical formulation according to any one of claims 1 to 10, comprising about 1004.60 mg of amoxycillin trihydrate and about 102.46 or 125.11 mg of potassium clavulanate.

13. A pharmaceutical formulation according to any one of claims 1 to 9 or 11, comprising about 1004.60 mg of amoxycillin trihydrate and about 109.41 mg of potassium clavulanate.

14. A pharmaceutical formulation according to any one of claims 1 to 13 comprising as effervescent couple sodium carbonate and an acid or acidic salt able to create carbon dioxide upon contact with an aqueous fluid.

15. A pharmaceutical tablet according to claim 14, wherein the acid is citric acid.

16. A pharmaceutical formulation according to any one of claims 1 to 15 wherein the buffer substance is an acidic salt.

17. A pharmaceutical formulation according to any one of claims 1 to 16, wherein the buffer substance is a mixture of citric acid monosodium citrate.

18. A pharmaceutical formulation according to any one of claims 1 to 17, comprising appropriate pharmaceutical excipients and coadjuvants selected from viscosity enlarging ingredients, fillers, desiccants, lubricants, diluents, binders, glidants, antifoaming agents, wetting agents, effervescent mixtures, sweeteners and flavourings.

19. A pharmaceutical formulation according to any one of claims 1 to 18, wherein the amoxycillin comprising granulate comprises as pharmaceutically acceptable ingredients microcrystalline cellulose, crosspovidone, povidone K25, collidone, colloidal anhydrous silica and optionally, a colouring agent, such as quinoline yellow.

20. A pharmaceutical formulation according to any one of claims 1 to 18, wherein the effervescent granulate comprises anhydrous citric acid, monosodium citrate, sorbitol, sodium hydrogen carbonate, a sweetener such as aspartam, povidone, Pharsil S 184, Tween 20, and a colouring agent, such as quinoline yellow.

21. A pharmaceutical formulation according to any one of claims 1 to 18, wherein the final blend comprises potassium clavulanate, microcrystalline cellulose, colloidal anhydrous silica, polyethylene glycol 6000, and the amoxycillin and effervescent granulates.

22. A pharmaceutical formulation according to any one of claims 1 to 21, of which the tablet manufactured thereof is disintegrating in aqueous fluids of room temperature within about 0.4 -1 minute.

23. A pharmaceutical formulation according to any one of claims 1 to 21, which is disintegrating in aqueous fluids of room temperature to result in a pH about 5.6 to 6.6, in particular about 6.

24. A method for the manufacture of a pharmaceutical formulation according to any one of claims 1 to 23, **characterised in**
a) preparing an amoxycillin comprising granulate by homogeneously mixing amoxycillin with the appropriate additional pharmaceutically acceptable excipients and coadjuvants, granulating the mixture, drying the granulates and passing the granulates through a suitable sieve,
b) preparing an effervescent granulate by homogeneously mixing the effervescence with the additional pharmaceutically acceptable excipients and coadjuvants, granulating the mixture, drying the granulates and passing the granulates through a suitable sieve, and
c) preparing a final blend by mixing the clavulanate with a predried mixture of the appropriate additional pharmaceutically acceptable excipients and coadjuvants, adding the amoxycillin granulate and then the effervescent granulate, and
d) compressing the mixture into tablets or filling into sachets or bottles.

25. A method of use of a pharmaceutical formulation as set forth in any one of claims 1 to 23, for the manufacture of a medicament for the treatment of bacterial infections.

26. A method of orally treating bacterial infections of a host, comprising administering to said host an aqueous fluid prepared from a pharmaceutical formulation according to any one of claims 1 to 25.
